# EUROPEAN PATENT SPECIFICATION

(11) **EP 3 305 263 B1**
(45) Date of publication and mention of the grant of the patent: **26.08.2020**
(21) Application number: 15894304.3
(22) Date of filing: 03.06.2015
(51) Int. Cl.: A61G 13/00, A61G 15/00, A61G 13/04, A61G 13/12, A61G 15/02, A61G 15/12, A47C 17/86, A61N 5/06, A61N 1/36, A61M 1/08, A61B 18/06

(54) **INTEGRATED BED FOR ORIENTAL MEDICINE THERAPY**
INTEGRIERTES BETT FÜR ORIENTALISCHE ARZNEIMITTELTHERAPIE
LIT INTÉGRÉ POUR THÉRAPIE PAR MÉDECINE ORIENTALE

(43) Date of publication of application: 11.04.2018
(73) Proprietor: Korea Institute of Oriental Medicine, Daejeon 305-811 (KR)
(72) Inventor: RYU, Yeon Hee, Daejeon 305-761 (KR); PARK, Ji Eun, Daejeon 305-761 (KR); KWON, O Sang, Iksan-si Jeollabuk-do 570-758 (KR)
(74) Representative: Gevers Patents
(86) International application number: PCT/KR2015/005557
(87) International publication number: WO 2016/195131

(56) References cited:
- EP-A2- 1 525 870
- JP-A- 2008 029 533
- KR-A- 20020 093 686
- KR-A- 20100 028 734
- KR-A- 20150 075 799
- KR-B1- 101 006 309
- KR-Y1- 200 248 076
- US-A- 4 746 167
- US-A1- 2013 019 402
- US-A1- 2013 307 298

## Description

### Technical Field

Embodiments of the present invention relate to an integrated bed for oriental medicine therapy, more particularly, to an integrated bed for oriental medicine therapy that may enable a patient to take various postures in comfort such that therapy may be performed in an optimized situation for the patient to receive the therapy, and that may be equipped with a therapy apparatus to improve an efficiency of space use.

### Background Art

In general, a patient lies down on a treatment bed to receive oriental medicine therapy. For example, the patient may receive therapy such as moxibustion, cupping, or acupuncture while lying down on the treatment bed.

However, the existing treatment bed has a fixed structure that simply provides a patient with only a room to lie down. The patient needs to take a posture suitable for receiving therapy while lying down on the treatment bed irrespective of a condition of the patient, which may cause discomfort to the patient.

For example, the patient needs to lie on an abdomen with a face down to receive cupping therapy on a body part of the patient, for example, a back. In this example, the patient may lie with a head turned to one side and placed on the treatment bed. Thus, when the cupping therapy is performed for a long time, the patient may feel discomfort or pain from excessive stress on a neck.

Accordingly, development of a bed for oriental medicine therapy having a new structure that may adjust an angle thereof for a patient to take a posture suitable for receiving therapy, thereby enabling the patient to feel comfort while receiving the therapy, is needed urgently.

EP 1 525 870 A2 discloses an integrated bed for oriental medicine therapy according to the preamble of claim 1.

### Disclosure of Invention

### Technical Goals

An aspect of the present invention provides an integrated bed for oriental medicine therapy that may enable a patient to take various postures for oriental medicine therapy and appropriately adjust positions of elements that support body parts of the patient, thereby enabling the oriental medicine therapy to be performed in an optimized situation for the patient to receive the therapy.

Another aspect of the present invention also provides an integrated bed for oriental medicine therapy that may easily adjust a slope angle of a movable bed body on which a patient lies down to receive oriental medicine therapy, thereby enabling the oriental medicine therapy to be performed in an optimized situation for the patient to receive the therapy.

Still another aspect of the present invention also provides an integrated bed for oriental medicine therapy that may be equipped with oriental medicine therapy apparatuses for treatment of a patient, thereby improving an efficiency of space use and simplifying a preparation process for the treatment.

Further another aspect of the present invention also provides an integrated bed for oriental medicine therapy that may include rests configured to rest a head portion, an arm portion, and a leg portion of a patient, thereby preventing an application of excessive stress to a predetermined part of the patient during therapy.

### Technical solutions

According to the present invention, there is provided an integrated bed for oriental medicine therapy on which oriental medicine therapy for a patient is performed, the integrated bed including a seat, a first support connected to one side of the seat so as to adjust a position thereof with respect to the seat, the first support configured to support a portion of a body of the patient, a second support connected to another side of the seat so as to adjust a position thereof with respect to the seat, the second support configured to support another portion of the body of the patient, and a compartment provided in at least one of the seat, the first support, and the second support, the compartment configured to store an oriental medicine therapy apparatus for treatment of the patient. By the above configuration, a patient may take various postures for oriental medicine therapy and positions of elements that support body parts of the patient may be adjusted appropriately, whereby the oriental medicine therapy may be performed in an optimized situation for the patient to receive the therapy. The first support includes a shoulder supporting member configured to support a shoulder portion of the patient, a head supporting member connected to the shoulder supporting member, the head supporting member configured to support a head of the patient, and a connecting member configured to connect the shoulder supporting member to the seat. The connecting member may be configured to tilt with respect to the seat, and the shoulder supporting member may be configured to tilt with respect to the connecting member. The second support includes a chest supporting member configured to support a chest portion of the patient, a chin supporting member connected to the chest supporting member, the chin supporting member configured to support a chin portion of the patient, and a movable connecting member movably coupled to a movable rail provided in the seat, the movable connecting member with one end to which the chest supporting member is tiltably coupled.

The integrated bed may further include a driver configured to generate a driving force to adjust the position of the first support or the second support with respect to the seat.

The integrated bed may further include an arm rest mounted on the seat so as to adjust a position thereof with respect to the seat, the arm rest configured to rest an arm of the patient, and a leg support mounted on the seat so as to adjust a position thereof with respect to the seat, the leg support configured to support a leg of the patient.

### Effects

According to embodiments, a patient may take various postures for oriental medicine therapy and positions of elements that support body parts of the patient may be adjusted appropriately, whereby the oriental medicine therapy may be performed in an optimized situation for the patient to receive the therapy.

According to embodiments, a slope angle of a movable bed body on which a patient lies down to receive oriental medicine therapy may be easily adjusted, whereby the oriental medicine therapy may be performed in an optimized situation for the patient to receive the therapy.

According to embodiments, oriental medicine therapy apparatuses for treatment of a patient may be stored, whereby an efficiency of space use may improve and a preparation process for the treatment may be simplified.

According to embodiments, rests configured to rest a head portion, an arm portion, and a leg portion of a patient may be provided, whereby an application of excessive stress to a predetermined part of the patient during therapy may be prevented.

### Brief Description of Drawings

FIG. 1 illustrates an integrated bed for oriental medicine therapy according to an embodiment.
FIG. 2 illustrates an example of adjusting a slope of a movable bed body with respect to a base bed body of FIG. 1.
FIG. 3 is a top view of the integrated bed for oriental medicine therapy of FIG. 1.
FIG. 4 illustrates elements to be disposed in a third space of the base bed body of FIG. 2.

### Best Mode for Carrying Out the Invention

Hereinafter, a configuration and an application will be described in detail with reference to the accompanying drawings. The following description will be one of aspects of the present disclosure and constitute a portion of the detailed description of the present disclosure.

However, when describing the present disclosure, detailed description related to a known function or configuration will be omitted for clarity.

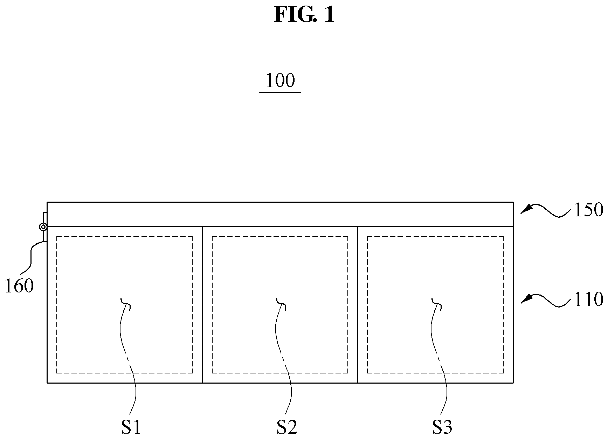

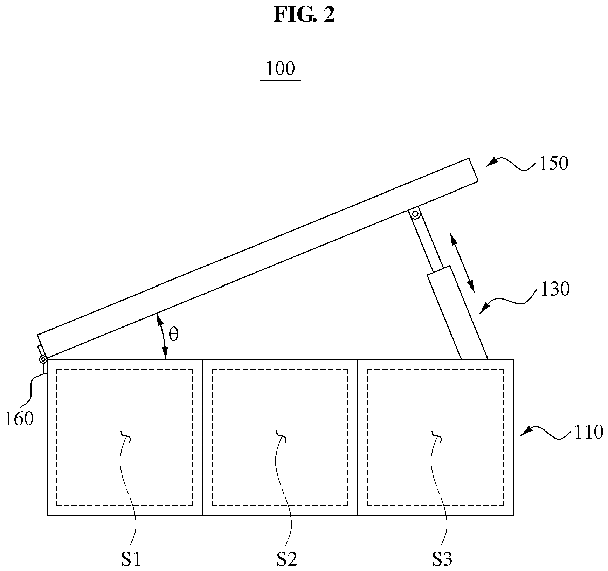

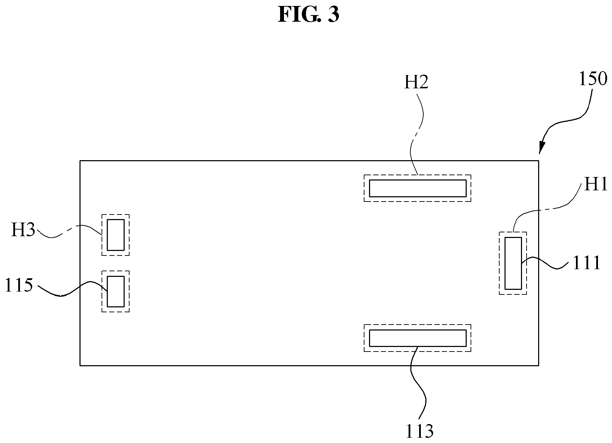

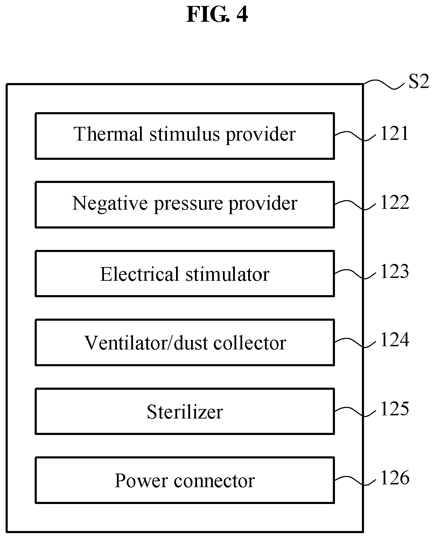

FIG. 1 illustrates an integrated bed for oriental medicine therapy according to an embodiment, FIG. 2 illustrates an example of adjusting a slope of a movable bed body with respect to a base bed body of FIG. 1, FIG. 3 is a top view of the integrated bed for oriental medicine therapy of FIG. 1, and FIG. 4 illustrates elements to be disposed in a third space of the base bed body of FIG. 2.

Referring to FIGS. 1 and 2, an integrated bed 100 for oriental medicine therapy is an integrated bed to be used for treatment of a patient at an oriental medicine clinic. The integrated bed 100 may include a base bed body 110 that is supported by the ground and forms a fundamental base of the integrated bed 100, a movable bed body 150 that is provided on a top of the base bed body 110 so as to adjust a slope thereof with respect to the base bed body 110, and a driver 130 configured to rotate the movable bed body 150 with respect to the base bed body 110.

By the above configuration, a patient may adjust the slope of the movable bed body 150, on which the patient actually lies down, with respect to the base bed body 110 based on a physical condition or body measurements of the patient, rather than receiving therapy while lying on an existing bed having a surface simply parallel to the ground. Thus, the patient may receive the therapy in comfort, and a therapist may treat the patient conveniently with a high precision.

The elements will be described further hereinafter. As shown in FIGS. 1 and 2, the base bed body 110 may be supported by the ground, and the top thereof may support the movable bed body 150. As described further below, the base bed body 110 may be partitioned into a plurality of spaces S1, S2, and S3 so as to be equipped with oriental medicine therapy apparatuses to be used for treatment, and also with a plurality of elements configured to maintain a posture of the patient.

The base bed body 110 may be partitioned into a total of three spaces S1, S2, and S3. Referring to FIGS. 1 and 2, a space positioned in a direction toward a head of the patient may be referred to as the first space S1, a space positioned in a direction toward feet of the patient may be referred to as the second space S2, and a space disposed between the first space S1 and the second space S2 may be referred to as the third space S2. However, the above naming is for ease of description, and the base bed body 110 may be partitioned into more than or fewer than three spaces.

First, a configuration of the first space S1 will be described. As shown in FIG. 3, a head rest 111 configured to rest a head of the patient during therapy and an arm rest 113 configured to rest an arm of the patient may be disposed in the first space S1 so as to move toward the movable bed body 150.

In detail, as shown in FIG. 3, the movable bed body 150 may include holes H1 and H2 through which the head rest 111 and the arm rest 113 may penetrate. The head rest 111 and the arm rest 113 may protrude through the holes H1 and H2 to stably rest the head and the arm of the patient.

Degrees of protrusion of the head rest 111 and the arm rest 113 with respect to the holes H1 and H2 may be adjusted based on a type of therapy for the patient or the body measurements of the patient.

In a case in which the patient lies down on the movable bed body 150 while facing a ceiling, the head rest 111 may protrude from the hole H1 so as to lift up the head of the patient slightly. Conversely, in a case in which the patient lies with the face down to receive cupping therapy, the head rest may be positioned at a height corresponding to a surface of the movable bed body 150 to rest the head of the patient, thereby preventing an application of stress to a neck.

Meanwhile, as shown in FIG. 3, a foot support 115 configured to support a foot of the patient may be movably provided in the second space S2. The foot support 115 may be disposed to protrude with respect to a hole H3 provided in the movable bed body 150.

As described below, the movable bed body 150 may adjust a slope thereof so as to slope with respect to the base bed body 110. In this example, the patient may slide downward by gravity. The foot support 115 may support the foot of the patient such that the patient may take a posture suitable for receiving the therapy on the movable bed body 150.

Meanwhile, the third space S2 of the base bed body 110 may be equipped with multiple oriental medicine therapy apparatuses to be used for treatment of the patient, and thus the oriental medicine therapy apparatuses may not need to be prepared separately for treatment of the patient. The above configuration may reduce a use of space for separate preparation of the oriental medicine therapy apparatuses, and enable the treatment of the patient to be performed without separate preparation, thereby securing speedy and efficient therapy.

As roughly shown in FIG. 4, the third space S2 may be equipped with the oriental medicine therapy apparatuses such as a thermal stimulus provider 121, a negative pressure provider 122, an electrical stimulator 123, a ventilator/dust collector 124, and a sterilizer 125. These oriental medicine therapy apparatuses may be selectively used by being connected to an external power supply (not shown) through a power connector 126.

First, the thermal stimulus provider 121 may provide the patient with a thermal stimulus to perform moxibustion or infrared lamp therapy. The negative pressure provider 122 may be a therapy apparatus configured to perform cupping on the patient, and may provide a negative pressure to a target area of the patient for cupping. The electrical stimulator 123 may apply an electric stimulus to a target area of the patient to perform physiotherapy.

The ventilator/dust collector 124 may perform ventilation to discharge smoke generated by moxibustion therapy outward, and collect impurities such as dust in a therapy site, thereby enabling a therapy procedure to be performed in a pleasant environment.

Further, the sterilizer 125 may sterilize an acupuncture needle used for oriental medicine therapy, thereby preventing contamination of the therapy apparatuses. In this example, ultraviolet ray sterilization may be applied as the sterilization. However, embodiments are not limited thereto.

However, the types of the oriental medicine therapy apparatuses are not limited thereto. Depending on purposes of oriental medicine therapy, other types of oriental medicine therapy apparatuses may be stored in the third space S2 of the base bed body 110.

Further, although it is described that the oriental medicine therapy apparatuses are installed in the third space S2, embodiments are not limited thereto. The oriental medicine therapy apparatuses may be installed in another space, for example, the first space S1 or the second space S2.

In the above example, the oriental medicine therapy apparatuses may be installed in the base bed body 110, rather than being separately installed outside of the therapy bed 100, whereby the efficiency of space use may improve and a preparation process for therapy may be simplified.

Meanwhile, as roughly shown in FIGS. 1 and 2, the movable bed body 150 may be coupled to the base bed body 110, in detail, a bottom of the movable bed body 150 may be coupled to the base bed body 110 through a hinge 160 such that the movable bed body 150 may rotate about the hinge 160.

As described above, the movable bed body 150 may include the plurality of holes HI, H2, and H3 through which the rests 111 and 113 provided in the first space S1 and the foot support 115 provided in the second space S2 may penetrate. Thus, the patient lying down on the movable bed body 150 may take a most comfortable posture to be suitable for the body measurements of the patient.

The slope of the movable bed body 150 may be adjusted with respect to the base bed body 110, which may be performed by the driver 130.

As shown in FIG. 2, the driver 130 may be a hydraulic motor, and thus selectively adjust a slope angle θ of the movable bed body 150 with respect to the base bed body 110 within a range of 0 degrees to 70 degrees. However, the slope angle of the movable bed body 150 is not limited thereto.

Thus, the therapist may adjust the slope angle of the movable bed body 150 to a slope angle suitable for performing therapy based on a type of the therapy for the patient. In addition, the patient may request the therapist to adjust the slope angle of the movable bed body based on a therapy area and the body measurements of the patient.

However, although it is described that the hydraulic motor is applicable as the driver 130, the type of the driver 130 is not limited thereto. Another type of the driver 130 that may selectively adjust the slope angle of the movable bed body 150 with respect to the base bed body 110 may also be applicable.

For example, a manual driver (not shown) including a plurality of gears to adjust the slope angle of the movable bed body 150 with respect to the base bed body 110 in a case in which the therapist rotates one of the gears using a handle may be applicable.

As described above, the angle of the treatment bed, that is, the movable bed body 150, on which the patient lies down for oriental medicine therapy, may be easily adjusted such that the patient may receive the oriental medicine therapy in an optimized situation. Further, the multiple oriental medicine therapy apparatuses described above may be disposed in the third space S3 of the base bed body 110, whereby the efficiency of space use may improve and a preparation process for therapy may be simplified.

In addition, the rests 111 and 113 may be provided to rest the head portion and the arm portion and the support 115 may be provided to support the foot of the patient, whereby an application of excessive stress to a predetermined part of the patient during the therapy may be prevented.

Meanwhile, a configuration of an integrated bed for oriental medicine therapy according to the invention will be described hereinafter. Description of the substantially the same portion as the integrated bed described above will be omitted for conciseness.

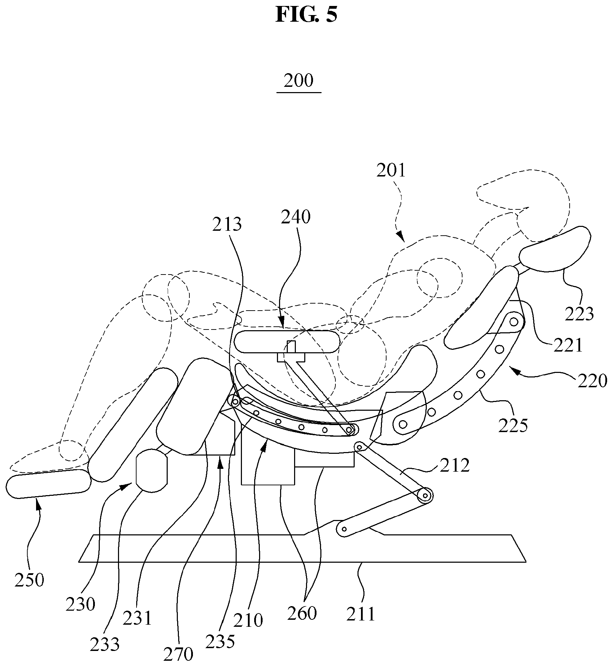

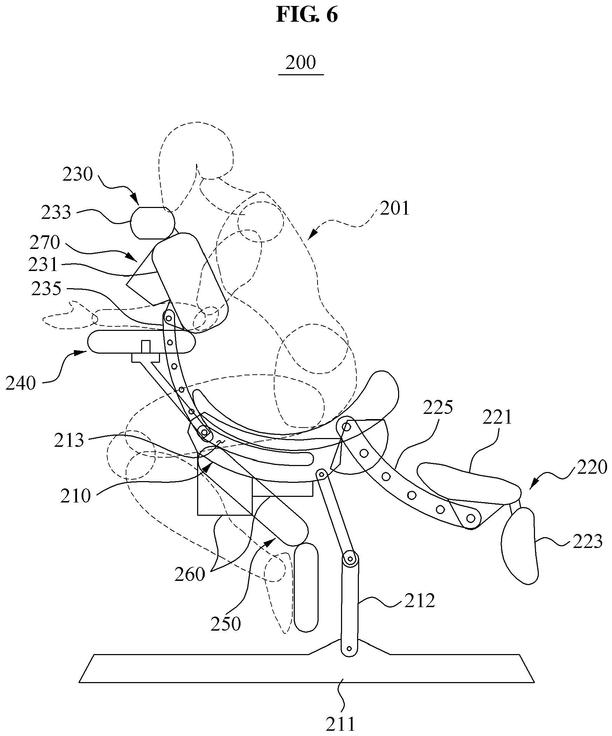

FIG. 5 illustrates a patient taking a first posture on an integrated bed according to another embodiment, and FIG. 6 illustrates the patient taking a second posture on the integrated bed of FIG. 5.

As shown in the drawings, an integrated bed 200 may include a seat 210 on which a patient 201 sits, a first support 220 tiltably connected to one side of the seat 210 and configured to support an upper body of the patient 201, and a second support 230 tiltably connected to another side of the seat 210 and configured to support the upper body of the patient 201 in another posture. The integrated bed 200 may further include an arm rest 240 mounted on the seat 210 so as to adjust a position thereof with respect to the seat 210 and configured to rest an arm of the patient 201, and a leg support 250 similarly mounted on the seat 210 so as to adjust a position thereof with respect to the seat 210 and configured to support a leg of the patient 201.

In addition, a plurality of compartments 260 partitioned from each other may be provided under the seat 210 such that oriental medicine therapy apparatuses may be stored therein. Thus, the oriental medicine therapy apparatuses may not need to be prepared separately for treatment of the patient 201, which will be described further below.

The elements will be described hereinafter. The seat 210 may be an element on which the patient 201 sits, and may partially support hips, a waist, and thighs of the patient 201. The seat 210 may be connected to a ground support plate 211 and a height adjuster 212, and a position of the seat 210 may be easily adjusted by controlling the height adjuster.

A plurality of elements may be mounted under the seat 210. A driver 270 configured to adjust positions of the first support 220 and the second support 230 may be mounted under the seat 210, and the plurality of compartments 260, for example, two compartments 260, configured to store the multiple oriental medicine therapy apparatuses may be mounted under the seat 210.

Various driving apparatuses may be used as the driver 270. For example, an air pump may be used. However, embodiments are not limited thereto.

As shown in FIG. 5, the first support 220 may include a shoulder supporting member 221 configured to support a shoulder portion of the upper body of the patient 201, a head supporting member 223 configured to support a head of the patient 201, and a connecting member 225 configured to connect the shoulder supporting member 221 and the seat 210.

Here, one end of the connecting member 225 may be tiltably coupled to one side of the seat 210 and another end of the connecting member 225 may be tiltably coupled to the shoulder supporting member 221, thereby appropriately adjusting the position of the first support 220 with respect to the seat 210.

For example, in a case in which the first support is positioned as shown in FIG. 5, the upper body of the patient 201 may be stably supported by the first support 220. In a case in which the patient 201 takes another posture as shown in FIG. 6, the first support 220 may be moved downward from the seat 210 to prevent interference with the patient 201.

Further, the head supporting member 223 may tilt with respect to the shoulder supporting member 221, thereby enabling the patient 201 to lean on the first support 220 in most comfort.

Meanwhile, as shown in FIGS. 5 and 6, the second support 230 may be coupled to the seat 210, have a structure that is movable along a movable rail 213 provided in the seat 210, and also have a tiltable structure.

The second support 230 may include a chest supporting member 231 configured to support a chest portion of the upper body of the patient 201, a chin supporting member 233 tiltably coupled to the chest supporting member 231 and configured to support a chin portion of the patient 201, and a movable connecting member 235 movably coupled to the movable rail 213 provided in the seat 210, the movable connecting member 235 with one end to which the chest supporting member 231 is tiltably coupled.

In a case in which the patient 201 takes the second posture as shown in FIG. 6, the upper body of the patient 201 may be stably supported by the above configuration. Thus, a therapist may easily perform therapy on a body part such as a back of the patient 201.

The positions of the first support 220 and the second support 230 may be adjusted by the driver 270. Although not shown in detail in the drawings, the driver 270 such as an air pump may move the movable connecting member 235 with respect to the movable rail 213, and thereby adjust an angle of the first support 220 or the second support 230. The positions of the first support 220 and the second support 230 may be adjusted by a driving force provided from the driver. However, embodiments are not limited thereto. The positions of the first support 220 and the second support 230 may be adjusted manually.

Meanwhile, the position of the arm rest 240 may be adjusted as shown in FIGS. 5 and 6. In a case in which the patient 201 takes the first posture of lying down as shown in FIG. 5, the arm rest 240 may be positioned above the seat 210 to stably rest the arm of the patient 201. In a case in which the patient 201 takes the second posture of sitting while leaning forward as shown in FIG. 6, the position of the arm rest 240 may be adjusted with respect to the seat 210 so as to stably rest the arm of the patient 201 leaning forward with respect to the first support 220. This may be achieved since the arm rest 240 has a structure that is tiltable with respect to the seat 210.

Meanwhile, as shown in FIGS. 5 and 6, the position of the leg support 250 may be adjusted with respect to the seat 210. In a case in which the patient 201 takes the first posture as shown in FIG. 5, the position of the leg support 250 may be maintained such that the patient 201 may stretch out the leg to slope downward. In a case in which the patient 201 takes the second posture as shown in FIG. 6, the position of the leg support 250 may be adjusted such that the patient 201 may bend the leg.

In the above example, the positions of the first support 220, the second support 230, the arm rest 240, and the leg support 250 may be adjusted with respect to the seat 210, whereby oriental medicine therapy may be performed in an optimized situation for the patient 201 to receive therapy.

Meanwhile, as described above, the oriental medicine therapy apparatuses such as a thermal stimulus provider, a negative pressure provider, an electrical stimulator, a ventilator/dust collector, and a sterilizer may be stored in the compartments 260. These oriental medicine therapy apparatuses may be selectively used by being connected to an external power supply through a power connector.

However, the types of the oriental medicine therapy apparatuses are not limited thereto. Other types of oriental medicine therapy apparatuses may be stored in spaces of the compartments 260 based on purposes of oriental medicine therapy.

As described above, in the above example, the oriental medicine therapy apparatuses may be installed in the compartments 260 mounted below the seat 210, rather than being separately installed outside of the integrated bed 200, whereby the efficiency of space use may improve and a preparation process for therapy may be simplified.

Meanwhile, although not described or illustrated in the above embodiments, a display device for monitoring may be mounted on an integrated bed such that a patient or a therapist may receive or perform therapy while viewing the display device together. A window containing information such as a therapy progress of the patient may be displayed on the display device, or an image to relieve a boredom during therapy may be provided. However, embodiments are not limited thereto.

The scope of the invention is defined by the appended claims.

## Claims

1. An integrated bed (200) for oriental medicine therapy on which oriental medicine therapy for a patient is performed, the integrated bed comprising:
a seat (210);
a first support (220) connected to one side of the seat (210) so as to adjust a position thereof with respect to the seat, the first support being configured to support a portion of a body of the patient;
a second support (230) connected to another side of the seat (210) so as to adjust a position thereof with respect to the seat, the second support being configured to support another portion of the body of the patient; and
a compartment (260) provided in at least one of: the seat (210), the first support (220), and the second support (230), the compartment being configured to store an oriental medicine therapy apparatus for treatment of the patient;
**characterized in that** the first support (220) comprises:
a shoulder supporting member (221) configured to support a shoulder portion of the patient;
a head supporting member (223) connected to the shoulder supporting member and configured to support a head of the patient; and
a connecting member (225) configured to connect the shoulder supporting member (221) to the seat (210) and being configured to tilt with respect to the seat, and the shoulder supporting member being configured to tilt with respect to the connecting member;
and **in that** the second support (230) comprises:
a chest supporting member (231) configured to support a chest portion of the patient;
a chin supporting member (233) connected to the chest supporting member (231), the chin supporting member configured to support a chin portion of the patient; and
a movable connecting member (235) movably coupled to a movable rail (213) provided in the seat (210) at one end thereof and tiltably coupled to the chest supporting member (231) at another end thereof.

2. The integrated bed of claim 1, further comprising:
a driver (270) configured to generate a driving force to adjust the position of one or more of the first support (220) and the second support (230) with respect to the seat (210).

3. The integrated bed of claim1, further comprising:
an arm rest (240) mounted on the seat (210) so as to adjust a position thereof with respect to the seat, the arm rest configured to rest an arm of the patient; and
a leg support (250) mounted on the seat (210) so as to adjust a position thereof with respect to the seat, the leg support configured to support a leg of the patient.

## Patentansprüche

1. Integriertes Bett (200) für orientalische Medizin-Therapie, auf dem orientalische Medizin-Therapie für einen Patienten durchgeführt wird, wobei das integrierte Bett Folgendes umfasst:
einen Sitz (210);
eine erste Stütze (220), die mit einer Seite des Sitzes (210) verbunden ist, um eine Position davon in Bezug auf den Sitz einzustellen, wobei die erste Stütze konfiguriert ist, um einen Abschnitt eines Körpers des Patienten zu stützen;
eine zweite Stütze (230), die mit einer anderen Seite des Sitzes (210) verbunden ist, um eine Position davon in Bezug auf den Sitz einzustellen, wobei die zweite Stütze konfiguriert ist, um einen anderen Abschnitt des Körpers des Patienten zu stützen; und
ein Fach (260), das in mindestens einem von: dem Sitz (210), der ersten Stütze (220) und der zweiten Stütze (230) bereitgestellt ist, wobei das Fach konfiguriert ist, um eine orientalische Medizin-Therapie-Einrichtung zur Behandlung des Patienten aufzubewahren;
**dadurch gekennzeichnet, dass** die erste Stütze (220) Folgendes umfasst:
ein Schulterstützelement (221), das konfiguriert ist, um einen Schulterabschnitt des Patienten zu stützen;
ein Kopfstützelement (223), das mit dem Schulterstützelement verbunden ist und konfiguriert ist, um einen Kopf des Patienten zu stützen; und
ein Verbindungselement (225), das konfiguriert ist, um das Schulterstützelement (221) mit dem Sitz (210) zu verbinden, und konfiguriert ist, um sich in Bezug auf den Sitz zu neigen, und wobei das Schulterstützelement konfiguriert ist, um sich in Bezug auf das Verbindungselement zu neigen;
und dadurch, dass die zweite Stütze (230) Folgendes umfasst:
ein Bruststützelement (231), das konfiguriert ist, um einen Brustabschnitt des Patienten zu stützen;
ein Kinnstützelement (233), das mit dem Bruststützelement (231) verbunden ist, wobei das Kinnstützelement konfiguriert ist, um einen Kinnabschnitt des Patienten zu stützen; und
ein bewegliches Verbindungselement (235), das an einem Ende davon mit einer in dem Sitz (210) bereitgestellten beweglichen Schiene (213) beweglich gekoppelt ist und an einem anderen Ende davon mit dem Bruststützelement (231) neigbar gekoppelt ist.

2. Integriertes Bett nach Anspruch 1, weiter umfassend:
einen Antrieb (270), der konfiguriert ist, um eine Antriebskraft zu erzeugen, um die Position einer oder mehrerer der ersten Stütze (220) und der zweiten Stütze (230) in Bezug auf den Sitz (210) einzustellen.

3. Integriertes Bett nach Anspruch 1, weiter umfassend:
eine Armlehne (240), die an dem Sitz (210) montiert ist, um eine Position davon in Bezug auf den Sitz einzustellen, wobei die Armlehne konfiguriert ist, um einen Arm des Patienten aufzulegen; und
eine Beinlehne (250), die an dem Sitz (210) montiert ist, um eine Position davon in Bezug auf den Sitz einzustellen, wobei die Beinlehne konfiguriert ist, um ein Bein des Patienten zu stützen.

## Revendications

1. Lit intégré (200) pour thérapie par médecine orientale sur lequel une thérapie par médecine orientale pour un patient est effectuée, le lit intégré comprenant :
un siège (210) ;
un premier support (220) relié à un côté du siège (210) de manière à ajuster une position de celui-ci par rapport au siège, le premier support étant configuré pour supporter une partie du corps du patient ;
un second support (230) relié à un autre côté du siège (210) de manière à ajuster une position de celui-ci par rapport au siège, le second support étant configuré pour supporter une autre partie du corps du patient ; et
un compartiment (260) situé dans au moins un parmi : le siège (210), le premier support (220) et le second support (230), le compartiment étant configuré pour stocker un appareil de thérapie par médecine orientale pour traitement du patient ;
**caractérisé en ce que** le premier support (220) comprend :
un élément de support d'épaule (221) configuré pour supporter une partie d'épaule du patient ;
un élément de support de tête (223) relié à l'élément de support d'épaule et configuré pour supporter une tête du patient ; et
un élément de liaison (225) configuré pour relier l'élément de support d'épaule (221) au siège (210) et étant configuré pour s'incliner par rapport au siège, et l'élément de support d'épaule étant configuré pour s'incliner par rapport à l'élément de liaison ;
et **en ce que** le second support (230) comprend :
un élément de support de poitrine (231) configuré pour supporter une partie de poitrine du patient ;
un élément de support de menton (233) relié à l'élément de support de poitrine (231), l'élément de support de menton étant configuré pour supporter une partie de menton du patient ; et
un élément de liaison mobile (235) accouplé de manière mobile à un rail mobile (213) situé dans le siège (210) au niveau d'une première extrémité de celui-ci et accouplé de manière inclinable à l'élément de support de poitrine (231) au niveau d'une autre extrémité de celui-ci.

2. Lit intégré selon la revendication 1, comprenant en outre :
un élément d'entraînement (270) configuré pour générer une force d'entraînement pour ajuster la position d'un ou plusieurs parmi le premier support (220) et le second support (230) par rapport au siège (210).

3. Lit intégré selon la revendication 1, comprenant en outre :
un repose-bras (240) monté sur le siège (210) de manière à ajuster une position de celui-ci par rapport au siège, le repose-bras étant configuré pour reposer un bras du patient ; et
un support de jambe (250) monté sur le siège (210) de manière à ajuster une position de celui-ci par rapport au siège, le support de jambe étant configuré pour supporter une jambe du patient.
